(19) Europäisches Patentamt

European Patent Office

Office européen des brevets

(11) **EP 1 611 844 A1**

(12) **EUROPEAN PATENT APPLICATION**
published in accordance with Art. 158(3) EPC

(43) Date of publication:
**04.01.2006 Bulletin 2006/01**

(51) Int Cl.:
*A61B 5/04* (1968.09)   *A61B 5/0245* (1990.01)
*B60K 28/06* (1985.01)

(21) Application number: **04720710.5**

(22) Date of filing: **15.03.2004**

(86) International application number:
**PCT/JP2004/003390**

(87) International publication number:
**WO 2004/089209 (21.10.2004 Gazette 2004/43)**

(84) Designated Contracting States:
**AT BE BG CH CY CZ DE DK EE ES FI FR GB GR
HU IE IT LI LU MC NL PL PT RO SE SI SK TR**
Designated Extension States:
**AL LT LV MK**

(30) Priority: **03.04.2003 JP 2003100454**

(71) Applicant: **Pioneer Corporation
Tokyo 153-8654 (JP)**

(72) Inventors:
• **YANAGIDAIRA, Masatoshi,
Pioneer Corporation
Tsurugashima-shi, Saitama 350-2201 (JP)**

• **YASUSHI, Mitsuo,
Pioneer Corporation
Tsurugashima-shi, Saitama 350-2201 (JP)**
• **SHIODA, Takehiko,
Pioneer Corporation
Tsurugashima-shi, Saitama 350-2201 (JP)**

(74) Representative: **Tappe, Hartmut et al
Böck, Tappe, Kirschner,
Patent- und Rechtsanwälte,
Kantstrasse 40
97074 Würzburg (DE)**

(54) **LIVING BODY INFORMATION DETECTING DEVICE, CONTACT MEMBER USED THEREFOR, AND LIVING BODY INFORMATION DETECTING MEMBER-USE PAINT**

(57)    The invention provides an apparatus for detecting biological information for accurately detecting biological information such as a heart rate from a driver of an automobile or the like.

The invention uses an apparatus for detecting biological information 10 including a control wheel 1 to be gripped by a driver of an automobile who is a subject of biological information, an electrode 41 on the right side and an electrode 42 on the left side that are provided in the control wheel 1, and an AC differential amplifier 6 that is connected to the electrodes 41 and 42. A sum of a resistance R1 between the electrode 41 on the right side and the AC differential amplifier 6 and electrode impedances r1 and C1 between a hand 51 of the driver gripping the control wheel 1 and the electrode 41 is set to 1/100 or less of an input impedance Z1 in the AC differential amplifier 6, and the same setting is performed for the electrode 42 on the left side, whereby the above-described subject is attained.

FIG. 1A

**(Cont. next page)**

# FIG. 1B

**Description**

Technical Field

The present invention relates to an apparatus for detecting biological information for detecting biological information such as a heart beat and a brain wave of a person who drives, steers, or pilots a vehicle, a ship, an airplane, or the like, a contact member used for the apparatus, and a paint for biological information detecting member used for the apparatus.

Background Art

In recent years, an apparatus for detecting biological information for learning a condition of a driver's health during driving of an automobile has been examined. According to such an apparatus for detecting biological information, for example, it is possible to detect a heart beat of a driver during driving of an automobile and obtain a clue for judging a physical condition of the driver such as presence or absence of sleepiness, fatigue, feeling of impatience, or arrhythmia on the basis of such a heart beat.

Further, the driver, an administrator of the automobile, or the like learns a change in the physical condition in this way, whereby the driver can take measures such as leaving the driving after that to another person or taking a break. As described above, the apparatus for detecting biological information can contribute to safety at the time of driving of an automobile or the like.

Here, as such an apparatus for detecting biological information, there is known one that is provided with electrodes in a steering wheel member of an automobile and detect biological information with these electrodes (e.g., see Patent Document 1).

Patent Document 1

Japanese Patent Laid-Open No. 11-225975 (paragraph 0020, paragraph 0026, etc.)

However, in the conventional apparatus for detecting biological information, there is inconvenience in that accuracy of a detection signal of biological information may be low.

More specifically, individual impedances and resistances may fluctuate depending upon a structure and a material of electrodes used for detection of the biological information. In addition, in an amplifier to which the electrodes are connected, a signal of a living body corresponding to biological information detected by the electrodes maybe distorted, or noise may be mixed in the signal. Further, if accuracy of a biological signal corresponding to the biological information is low, there is inconvenient in that an accurate physical condition of a driver cannot be detected and the essential role of the apparatus for detecting biological information of contributing to safety at the time of driving cannot be accomplished.

Disclosure of the Invention

Therefore, an example of an object of this application is to solve the above-described inconvenience. And it is an object of this application to provide an apparatus for detecting biological information that can improve detection accuracy of a biological signal corresponding to biological information and contribute to safety at the time of driving of a vehicle or the like, a contact member used for the apparatus, and a paint for a biological information detecting member for the apparatus.

The above obj ect of the present invention can be achieved by an invention described in claim 1. The invention is an apparatus for detecting biological information comprising: a contact member arranged to come into contact with a subject of biological information; a biological information detecting member provided in the contact member and detects the biological information from the subject; and an amplifier connected to the biological information detecting member and amplifies a biological signal corresponding to the detected biological information, wherein a sum of a resistance between the biological information detecting member and the amplifier, and an impedance between the subj ect in contact with the contact member and the biological information detecting member, is not more than 1/100 of an input impedance in the amplifier.

The above obj ect of the present invention can be achieved by an invention described in claim 18. The invention is a contact member that is used as the contact member included in the apparatus for detecting biological information according to any one of claims 1 to 17, wherein the biological information detecting member has a resistance of not more than 5 kΩ (kohm).

The above obj ect of the present invention can be achieved by an invention described in claim 28. The invention is a paint for a biological information detecting member that constitutes a detecting member for detecting biological information from a subject, wherein the paint for a biological information detecting member comprises a conductive material having a volume resistivity of not more than 25 Ωcm (ohm centimeter), epoxy resin, and a curing agent.

Brief Description of Drawings

FIG. 1 is a diagram schematically showing an apparatus for detecting biological information according to an embodiment of the invention and an equivalent circuit thereof;

FIG. 2 is a diagram showing a portion where electrodes are formed in a control wheel in FIG. 1;

FIG. 3 is a diagram showing a structure for transmitting biological information of the apparatus according to one embodiment of the invention;

FIG. 4 is a diagram schematically showing an apparatus for detecting biological information according to another embodiment of the invention;

FIG. 5 is a diagram schematically showing an apparatus for detecting biological information according to still another embodiment of the invention; and

FIG. 6 is a diagram showing electrocardiographic complexes of an example and comparative examples.

(Explanation of symbols)

| 1 | Control wheel |
|---|---|
| 2 | Steering wheel |
| 3 | Spoke |
| 41, 42, 72, 82 | Electrode |
| 51, 52 | Man's hand |
| 6 | AC differential amplifier |
| 7 | Handbrake piece |
| 8 | Armrest piece |
| 10, 11, 12 | Apparatus for detecting biological information |
| Va1, Va2 | Potentials generated in pulsation of a heart (Initial potential) |
| Vb1, Vb2 | Potentials inputted to an amplifier |
| R1, R2 | Electrode-amplifier resistance |
| r1, r2 | Electrode impedance (part of resistance) |
| C1, C2 | Electrode impedance (part of capacitance) |
| Z1, Z2 | Input impedance of an amplifier |

Best Mode for Carrying Out the Invention

Next, preferred embodiments of the invention will be explained on the basis of the drawings.

Note that an apparatus for detecting biological information of the embodiments to be explained below is constituted by a control wheel of an automobile serving as a contact member arranged to be in contact with a subject of biological information (The subject is a man who is measured biological information.), electrodes serving as a biological information detecting member provided in this control wheel, and an amplifier serving as an amplifier to be connected to these electrodes. And the apparatus for detecting biological information sets a driver of the automobile as the subject of biological information, and outputs an electrocardiographic complex in order to detect a heart rate of the driver as biological information.

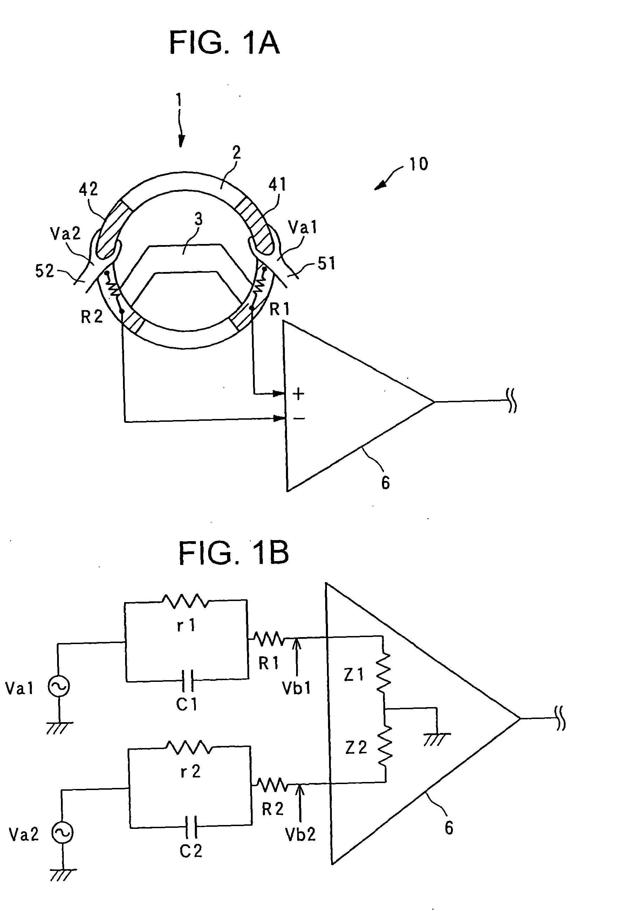

In the first place, a structure of the apparatus for detecting biological information according to an embodiment will be explained using FIG. 1.

As shown in FIG. 1(a), an apparatus for detecting biological information 10 of the embodiment includes a control wheel 1, electrodes 4, and an AC differential amplifier 6.

In this case, the control wheel 1 is constituted by a steering wheel 2 and a spoke 3 supporting this steering wheel 2. In the control wheel 1, on both left and right sides of the steering wheel 2, a pair of electrodes (an electrode 41 on the right side and an electrode 42 on the left side) are formed on the surface of the steering wheel 2 to detect a heart beat of a driver as biological information (in other words, a potential in hands of the driver). Further, the electrodes 41 and 42 are connected to the AC differential amplifier 6 (the AC differential amplifier 6 will be hereinafter also referred to simply as the amplifier 6) that detects a potential difference between the electrodes 41 and 42. Note that, in this application, the left and the right means the left and the right in a state of use of the member or the like.

Next, an electric equivalent circuit of a structure shown in FIG. 1 (a) will be explained using FIG. 1 (a) and FIG. 1 (b). In an equivalent circuit shown in FIG. 1(b), reference signs Va1 and Va2 denote potentials that are generated in both left hand and right hands according to pulsation of a heart (Va1 and Va2 will be hereinafter referred to as an initial potential Va). On the other hand, reference signs R1 and R2 shown in FIG. 1 (a) denote values of resistances between the electrodes 41 and 42 and the amplifier 6. R1 indicates a resistance from a position where a hand 51 of the driver is in contact with the electrode 41 of the control wheel 1 to the amplifier 6 (R1 and R2 will be hereinafter referred to as an electrode-amplifier resistance R). Here, the electrode-amplifier resistance R is a resistance depending upon a material of the electrodes 41 and 42, a distance from the amplifier 6 to the electrodes 41 and 42, lengths from positions where the hands of the driver are in contact with the electrodes 41 and 42 to ends of the electrodes 41 and 42, and a width and a thickness of the electrodes 41 and 42, and the like. The electrode-amplifier resistance R will be described in detail later.

Next, reference signs r1 and C1 as well as r2 and C2 denote impedances between hands 51 and 52 of the driver and the electrodes 41 and 42, respectively, which are called electrode impedances or contact impedances (hereinafter referred to as electrode impedances r and C in this application). Note that r indicates resistance components present between the hands 51 and 52 of the driver and the electrodes 41 and 42, and C indicates capacitance components present between the hands 51 and 52 of the driver and the electrodes 41 and 42. In addition, reference signs Z1 and Z2 denote input impedances in the amplifier 6 at respective input terminals.

Here, an electrocardiographic complex is obtained if predetermined amplification is applied to a potential difference between the Va1 obtained from the electrode 41 and the potential Va2 obtained from the electrode 42.

That is, first, when the electrode 41 on the right side is considered, naturally, it is desirable that the initial potential Va1 is directly obtained as an input value of the amplifier 6. However, actually, a value Vb1 obtained by dividing between 'a sum of the electrode-amplifier resistance R1 and the electrode impedances r1 and C1' and 'the input impedance Z1 of the amplifier' is an input value of the amplifier 6.

Similarly, in the electrode 42 on the left side, a value Vb2 obtained by dividing between 'a sum of the electrode-amplifier resistance R2 and the electrode impedances r2 and C2' and 'the input impedance Z2 of the amplifier' is an input value of the amplifier 6.

Further, in this embodiment, a sum of the electrode-amplifier resistance R (R1 or R2) and the electrode impedances r and C (r1 and C1 or r2 and C2) is set to 1/100 or less of the input impedance Z (Z1 or Z2) of the amplifier 6. More specifically, a sum of the electrode-amplifier resistance R1 and the electrode impedances r1 and C1 is set to 1/100 or less of the input impedance Z1 of the amplifier. And a sum of the electrode-amplifier resistance R2 and the electrode impedances r2 and C2 is set to 1/100 or less of the input impedance Z2 of the amplifier. Note that a lower limit value of the sum of the electrode-amplifier resistance R (R1 or R2) and the electrode impedances r and C ('r1 and C1' or 'r2 and C2') is not specifically limited but is set to 1/10000 or more of the input impedance Z (Z1 or Z2) of the amplifier 6.

In this case, by setting the sum of the electrode-amplifier resistance R and the electrode impedances r and C to such a range, it is possible to prevent the original signal (initial potential) Va1 or Va2 from being distorted and prevent noise from being mixed in a (+) input section and a (-) input section of the amplifier 6 due to fluctuation in respective impedances or resistances or due to an electrode impedance having frequency characteristics. As a result, an accurate electrocardiographic complex can be obtained from the amplifier 6.

Here, the input impedance Z of the amplifier 6 is usually 1 MΩ or more, the sum of the electrode-amplifier resistance R and the electrode impedances r and C is set to 10 kΩ (kohm) or less. By setting the sum of the electrode-amplifier resistance R and the electrode impedances r and C to such a range, as described above, the sum of the electrode-amplifier resistance R and the electrode impedances r and C is 1/100 or less of the input impedance Z of the amplifier. More specifically, the sum of the resistance R1 and the electrode impedance r1 and C1 is set to 10 kΩ or less, and the sum of the resistance R2 and the electrode impedances r2 and C2 is also set to 10 kΩ or less. Note that a lower limit value of the sum of the electrode-amplifier resistance R and the electrode impedances r and C is not specifically limited but is usually about 0.1 kΩ. This lower limit value will be described later.

In this case, if the sum of the resistance R and the electrode impedances r and C is within the above-described range, respective values of the electrode-amplifier resistance R and the electrode impedances r and C are not specifically limited but are usually set as described below.

That is, the electrode-amplifier resistance R is set to 1/200 or less of the input impedance Z of the amplifier 6. More specifically, the resistance R1 is set to 1/200 or less of the input impedance Z1 of the amplifier 6, and the resistance R2 is also set to 1/200 or less of the input impedance Z2 of the amplifier 6. Note that a lower limit value of the electrode-amplifier resistance R is not specifically limited but is usually set to $1/(2.3 \times 10^5)$ or more of the input impedance Z of the amplifier 6.

Further, since the input impedance Z of the amplifier 6 is usually 1 MΩ (mega ohm) or more as described above; the electrode-amplifier resistance R is set to 5 kΩ or less. By setting the electrode-amplifier resistance R to such a range, as described above, the electrode-amplifier resistance R is 1/200 or less of the input impedance Z of the amplifier 6. More specifically, the resistances R1 and R2 are set to 5 kΩ or less, respectively.

Here, in order to set the electrode-amplifier resistance R to 5 kΩ or less as described above, conductive resin or the like having a resistance of 5 kΩ or less is used for the electrodes 41 and 42. As a material (conductive component) for such conductive resin, a material having a volume resistivity $\rho$ of 25 $\Omega$cm or less can be used. As such a material, a material containing at least one of silver, nickel, gold, palladium, carbon, and carbon nanotube is preferably used. Note that volume resistivities of the respective materials is as follows: silver (Ag); $1.1\times10^{-4}$ $\Omega$cm, nickel (Ni); $2.7\times10^{-1}$ $\Omega$cm, gold (Au); $2.1\times10^{-2}$ $\Omega$cm, palladium (Pd); $8.2\times10^{-2}$ $\Omega$cm, carbon (C) ; $1.3\times10^{-1}$ $\Omega$cm. Since silver has the lowest volume resistivity among these materials, silver is preferably used as a high-performance conductive material for the electrodes 41 and 42. A lower limit value of the volume resistivity $\rho$ of the material of the conductive component is not specifically limited but is usually set to $1.1\times10^{-4}$ $\Omega$cm, which is the volume resistivity $\rho$ of silver.

In addition, as the conductive material, materials containing at least one of metal oxide, which is transparent and has electrical conductivity, and polymer, which is transparent and has electrical conductivity, are preferably used. Since the materials containing metal oxide or polymer have electrical conductivity, the electrode-amplifier resistance R can be set to 5 k$\Omega$ or less as described above. Moreover, since these materials are transparent, when a conductive resin layer 45 is formed with the materials contained in paint to be described later, a color of a member in which the conductive resin layer 45 is formed appears. Thus, the color of the member in which the conductive resin layer 45 is formed and a member around this member, for example, an interior of a vehicle can be matched. Consequently, a design property can be improved compared with the above-described conductive component such as silver.

Here, as metal oxide that is transparent and has electrical conductivity, for example, there are ITO (Indium Tin Oxide), ATO (Antimony Tin Oxide), AZO (Aluminum Zinc Oxide), and the like. Note that the volume resistivities $\rho$ of these materials are as follows: ITO; $9\times10^{-3}$ $\Omega$cm, ATO; $5\times10^{-3}$ $\Omega$cm, AZO; $2\times10^{-4}$ $\Omega$cm. All of the volume resistivities $\rho$ are equal to or less than 25 $\Omega$cm described above. In addition, as polymer that is transparent and has electrical conductivity, there is polymer generally called conductive polymer, for example, polyacetylene, polypyrrole, polythiophene, and the like doped with an electron acceptor or an electron donor. The conductive polymer is polymer having an electrical conductivity $10^{-7}$ S $\cdot$ cm$^{-1}$ or more.

In this embodiment, the electrodes 41 and 42 are formed as the conductive resin layer 45 on the steering wheel 2 of the control wheel 1. A paint forming this conductive resin layer 45 contains at least one of silver, nickel, gold, palladium, carbon, and carbon nanotube, which are used as a material for the electrodes 41 and 42, as a conductive component. And the paint contains epoxy resin or the like as a binder component, and further contains a curing agent. The above-described conductive component is contained in this paint at a percentage by mass of about 70 to 80. Note that, as the conductive component contained in this paint, considering a problem of deterioration in quality such as peeling-off and occurrence of crack after coating, silver is most stable and is used preferably.

Moreover, in order to form the conductive resin layer 45, the paint containing the conductive component is coated on the surface of the control wheel 1. An area and a thickness of this coating are usually set to a length of about 50 cm, a width of about 5 cm, and a thickness of about 50 $\mu$m (micrometer) according to a size of the control wheel 1. As described above, by setting the volume resistivity $\rho$ of the conductive component to 25 $\Omega$cm or less as described above, a resistance of a coating surface (i.e., the electrodes 41 and 42) can be set to 5 k$\Omega$ or less as indicated by the following expression (1).

$$5k\Omega > \rho \text{ x } 50(cm) \text{ / } (5(cm) \text{ x } 50(\mu m)) \qquad (1)$$

(according to Formula; resistance = $\rho$ x length / (width x thickness))

Note that, in accordance with expression (1), if a value of ($\rho$ x length / cross sectional area) is smaller than 5 k$\Omega$, an area (length and width) and a thickness of the electrodes 41 and 42 are not specifically limited. Here, a lower limit value of the resistance R is not specifically limited. However, if silver with the lowest volume resistivity $\rho$ is used and a length from a contact portion to a coating surface end is about 1 cm at the minimum, the resistance R is $4.4\times10^{-3}$ $\Omega$ or more.

On the other hand, the electrode impedances r and C are set to 1/200 or less of the input impedance Z in the amplifier 6. More specifically, the electrode impedances r1 and C1 are set to 1/200 or less of the input impedance Z1 in the amplifier 6, and the electrode impedances r2 and C2 are also set to 1/200 or less of the input impedance Z2 in the amplifier 6. Note that a lower limit value of the electrode impedances r and C is not specifically limited but is usually set to 1/10000 or more of the input impedance Z in the amplifier 6.

Here, since the input impedance Z of the amplifier 6 is usually 1 M$\Omega$ or more, the electrode impedances r and C are 5 k$\Omega$ or less. By setting the electrode impedances r and C to such a range, as described above, the electrode impedances rand Care 1/200 or less of the input impedance Z of the amplifier 6. More specifically, the electrode impedances r1 and C1 are set to 5 k$\Omega$ or less, and the electrode impedances r2 and C2 are also set to 5 k$\Omega$ or less.

Further, in order to set the electrode impedances r and C to 5 k$\Omega$ or less, the electrodes 41 and 42 are formed such that, for each of the left and the right of the control wheel 1, contact areas of the hands 51 and 52 of the driver and the electrodes 41 and 42 are 2 cm$^2$ or more for each place. Here, the electrode impedances r and C have frequency characteristics as described above and the electrode impedances increase as a frequency decreases. For example, in the case in which silver is used as a conductive component for the electrodes 41 and 42, considering that a lower limit of a frequency component of an electrocardiogram is around 1 Hz, an electrode impedance with respect to 1 Hz is about 10 KΩ (more specifically, for example, see Noha Denkyoku Pocket Chishiki <Denkyoku to Paste no Ohanashi> (June 9, 1993, NEC Sanei) p16). This value is a value in the case in which electrodes with an area of about 1 cm$^2$ used for medical purposes are used. However, if the contact area is increased, since r decreases and a capacitive impedance formed by C also decreases, the electrode impedances r and C decrease. Thus, by setting the contact area of the electrodes 4 formed in the control wheel 1 and the hands 5 of the driver to 2 cm$^2$ or more, the electrode impedances r and C can be lowered to 1/2 or less of 10 kΩ, that is, 5 kΩ or less. Moreover, by setting this contact area to 25 cm$^2$ or more, the electrode impedances r and C can be lowered to 1/25 or less of 10 kΩ, that is, about 0.4 kΩ. Furthermore, considering a state in which the entire palms of the driver are in contact with the electrodes 4, by setting this contact area to 100 cm$^2$ or less, the electrode impedances r and C can be lowered to 1/100 or less of 10 kΩ, that is, about 0.1 kΩ. Therefore, a lower limit value of impedances (electrode impedances r and C) between a subject and a biological information detecting member is not specifically limited but is usually about 0.1 kΩ.

From the above, the lower limit value of the sum of the resistance R and the electrode impedances r and C is set to 0.1 kΩ or more as described above and is set to a sum of the lower limit value of the resistance R and the lower limit value of the electrode impedances r and C. However, the lower limit value of the sum of the resistance R and the electrode impedances r and C is substantially equal to the lower limit value of the electrode impedances r and C.

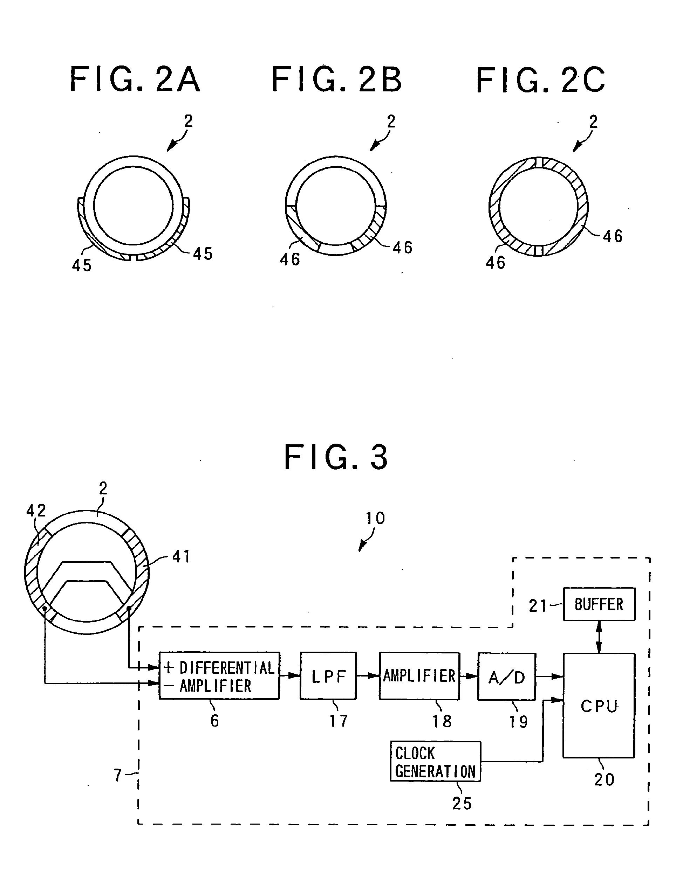

In order to obtain such a structure, for example, as shown in FIG. 2(a), the conductive resin layer 45 is formed as the electrodes 41 and 42 on the entire lower surface area not facing the driver. And the electrode 41 on the right side and the electrode 42 on the left side are not in contact with each other. Note that FIG. 2 is a diagram showing portions where electrodes are formed in the steering wheel 2 of the control wheel 1.

Next, a flow after potential information obtained by such an apparatus for detecting biological information 10 of this embodiment is outputted from the AC differential amplifier 6 will be explained using FIG. 3. FIG. 3 is a block diagram showing an example of a specific structure of the apparatus for detecting biological information 10 of this embodiment.

As shown in FIG. 3, the apparatus for detecting biological information 10 of the embodiment includes a heart beat data transmission apparatus 7 comprising the AC differential amplifier 6 that detects a potential difference between the electrodes 41 and 42, a Low Pass Filter 17 that removes an unnecessary noise component from an output signal of the AC differential amplifier 6, an amplifier 18 that amplifies an output of the LPF 17, an A/D (Analog/Digital) converter 19 that converts a heart beat signal from the amplifier 18 into a digital signal, a CPU 20 that performs an operation and control of the heart beat data transmission apparatus 7 with the digital heart beat signal from the A/D converter 19 as input data, a buffer 21 that temporarily stores the converted digital heart beat signal, and a clock generator 25 that gives an operation clock to the CPU 20.

Next, operations of the apparatus for detecting biological information 10 will be explained using FIGS. 1 to 3.

First of all, when the driver grips the steering wheel 2 with the hands 51 and 52 in order to drive the vehicle, both the hands of the driver come into contact with the pair of electrodes 41 and 42 mounted on the surface of the steering wheel 2. In this case, the electrodes 41 and 42 constitute a potential type heart beat sensor. The potential type heart beat sensor detects a pulse-like potential (myocardial action potential) between both the hands of the driver, which is generated according to electric excitation of a heart, from the electrodes 41 and 42 and detects beating of the heart. Then, the potential detected by the electrodes 41 and 42 is inputted to the AC differential amplifier 6, and amplifier 6 detects a potential difference between the electrodes 41 and 42. Next, an output of the AC differential amplifier 6 is inputted to the LPF 17, various kinds of noise such as noise caused by the vehicle are removed from the output, and only a 20 Hz heart beat signal component (about 20 Hz) of the output is passed. Then, the heart beat signal, which has passed the LPF 17, is amplified to a predetermined level by the amplifier 18. Then, the heart beat signal, which has passed the LPF 17, is converted into a digital signal by the A/D converter 19. Thereafter, a waveform of this digital heart beat signal, that is, digital myocardial active potential data is inputted to the CPU 20. And the date is inputted to the buffer 21 from the CPU 20, and stored in the buffer 21 temporarily. At this point, information indicating time when the digital heart beat data is inputted to the CUP 20, that is, information indicating at what time the heart beat of the driver is detected can also be stored. Note that an operation clock is supplied to the CPU 20 by the clock generator 25.

An electrocardiographic complex is obtained by, for example, amplifying a potential, which is generated by a heart beat of the hands of the driver, detected by the electrodes 41 and 42 in this way according to circumstances.

Note that data of the potential detected by the electrodes 41 and 42 can be used as described below. For example, it is possible to transmit biological information during driving by the driver to another apparatus on a real time basis, diagnose a physical condition of the driver with the apparatus, and if the physical condition is diagnosed as abnormal, immediately warn the driver of the abnormality. In addition, it is also possible that the biological information during the driving is stored in a predetermined apparatus, and after the driver ends the driving, the driver can be informed of the biological information of the driver during the driving. In this way, as an apparatus for informing the driver of the biological information to the driver, a car navigation apparatus is preferably used.

As explained above, according to the apparatus for detecting biological information 10 of this embodiment, since the sum of the electrode-amplifier resistance R and the electrode impedances r and C is 1/100 or less of the input impedance Z in the amplifier 6, a biological (potential) signal of the hands, which is generated according to beating of the heart of the driver as biological information, is prevented from being distorted, and noise is prevented from being mixed in the biological signal. Therefore, an accurate potential can be amplified and outputted in the amplifier 6, and an accurate electrocardiographic complex can be obtained. In this way, according to the apparatus for detecting biological information 10 of this embodiment, it becomes possible to perform detection of a heart beat of the driver stably even at the time of driving.

In addition, according to the control wheel 1 of the automobile used in this embodiment, since the electrode 41 has a resistance of 5 k$\Omega$ or less, the resistance R1 between the electrode 41 and the amplifier 6 is 5 k$\Omega$ or less. Further, since the electrode 42 has a resistance of 5 k$\Omega$ or less, the resistance R2 between the electrode 42 and the amplifier 6 is 5 k$\Omega$ or less. These resistances R1 and R2 are 1/200 or less of the input impedance Z1 or Z2 of the amplifier 6, which is usually about 1 M$\Omega$, respectively. Therefore, the sum of 'the electrode-amplifier resistance R' and 'the electrode impedances r and C' can be set to 1/100 or less of the input impedance Z in the amplifier 6. Consequently, as biological information, a potential signal of the hands, which is generated according to beating of the heart of the driver, is prevented from being distorted in the input section of the amplifier 6, and noise is prevented from being mixed in the potential signal. Therefore, an accurate potential can be amplified and outputted in the amplifier 6, and an accurate electrocardiographic complex can be obtained. In this way, according to the apparatus for detecting biological information 10 of this embodiment, it becomes possible to perform detection of a heart beat of the driver stably even at the time of driving.

Further, according to the paint for electrode formation used in this embodiment, since the paint is constituted by a conductive material with the volume resistivity $\rho$ of 25 $\Omega$cm or less, for example, in the electrodes 41 and 42 formed by coating this paint with an area of about 250 cm$^2$ (5 cmx50 cm) and a thickness of about 50 $\mu$m, a resistance of the electrodes 41 and 42 can be set to 5 k$\Omega$ or less. Therefore, the resistance R between the electrode 41 (or 42) and the amplifier 6 is 5 k$\Omega$ or less. This resistance R is 1/200 or less of the input impedance Z of the amplifier 6 that is usually about 1 M$\Omega$. Therefore, the sum of the electrode-amplifier resistance R and the electrode impedances r and C can be set to 1/100 or less of the input impedance Z in the amplifier 6. Consequently, a biological signal of the hands, which is generated according to beating of the heart of the driver as biological information, is prevented from being distorted in the input section of the amplifier 6, and noise is prevented from being mixed in the biological signal. Therefore, an accurate potential can be amplified and outputted in the amplifier 6, and an accurate electrocardiogram can be obtained. In this way, according to the apparatus for detecting biological information, it becomes possible to perform detection of a heart beat of the driver stably even at the time of driving.

Next, a modification of the above-described embodiment of the apparatus for detecting biological information 10 will be explained.

In the above-described embodiment of the apparatus for detecting biological information 10, the control wheel 1 of the automobile is explained as the member with which a subject of biological information comes in contact. However, the member is not limited to this, and this application can be applied to a controller of any one of vehicles such as an automobile, a ship, and an airplane. In addition, the member may be a member other than the control wheel as long as a person can come into contact with or grip the member. For example, a throttle lever, a switch used for actuation and control of a machine or an apparatus, or the like can be used.

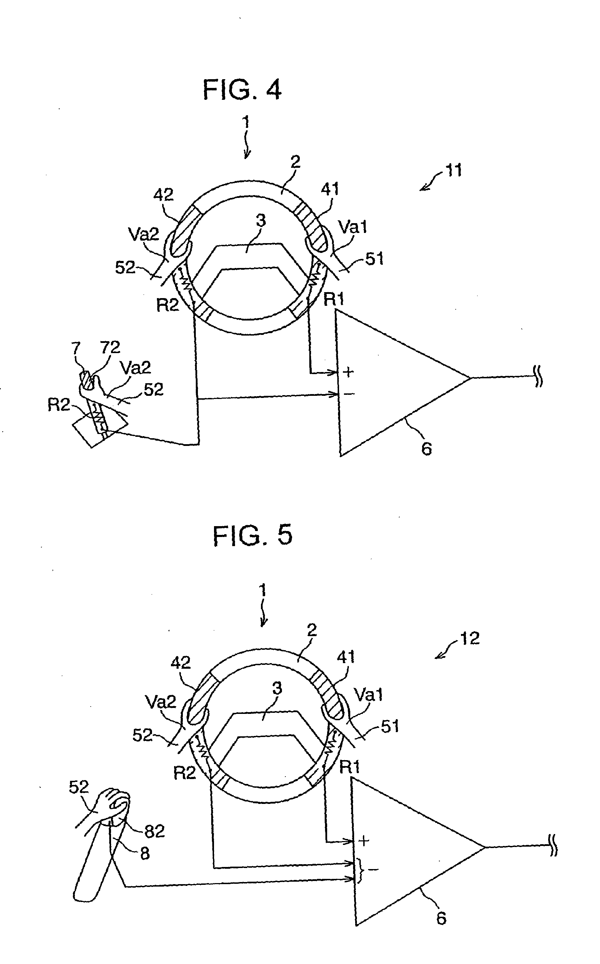

Moreover, the member with which the subject of biological information comes into contact may take a form comprising a controller used for controlling at least one of an automobile, a ship, and an airplane, and an auxiliary contact piece that is constituted to assist the subj ect when the subj ect controlling the vehicle using this controller comes into contact with the auxiliary contact piece. This form will be explained using apparatuses for detecting biological information 11 and 12 shown in FIGS. 4 and 5. Note that, in FIGS. 4 and 5, members having the same functions as those of the members shown in FIG. 1 are denoted by the same reference numerals and signs, and an explanation of the members will be omitted.

When the apparatus for detecting biological information is provided in an automobile, the auxiliary contact piece is, for example, a side brake piece 7 used as an auxiliary brake as shown in FIG. 4, an armrest piece 8 that is used for resting an arm as shown in FIG. 5, or a shift lever piece that is used for selecting a function such as parking or driving in the case in which a transmission of the automobile is an automatic type (so-called automatic car) or used for switching a transmission of the automobile in the case in which the transmission is a manual type (so-called manual car). In addition, as the auxiliary contact piece, a member or the like that assists control when the subject controls an automobile or the like or assisting a body of the subject. At least one of these members is used as the auxiliary contact piece.

In addition, as shown in FIG. 4, the apparatus for detecting biological information 11 can be constituted such that the electrode 42 serving as the biological information detecting member, which is provided in the steering wheel 1 serving as the controller, and an electrode 72 serving as a biological information detecting member, which is provided in the side brake piece 7 serving as the auxiliary contact piece, are connected in the electrodes side of the amplifier 6 serving as the amplifier. Note that the electrode 42 and the electrode 72 may be short-circuited.

With such a structure, the amplifier 6 can detect a biological signal of one of the electrode 42 and the electrode 72, with which the subject is in contact, and amplifies this signal to obtain an expected effect. Note that, in FIG. 4, the apparatus for detecting biological information 11 is constituted such that only the electrodes (-) 42 and 72 on the left hand side are connected. However, the apparatus for detecting biological information 11 may be constituted in the same manner by also providing an auxiliary contact piece for the electrode (+) 41 on the right hand side.

Moreover, as shown in FIG. 5, the apparatus for detecting biological information 12 can be constituted such that the amplifier 6 serving as the amplifier amplifies a biological signal detected by one of the electrode 42 provided in the steering wheel 1 and an electrode 82 serving as a biological information detecting member that is provided in the armrest piece 8 serving as the auxiliary contact piece, with which the subject is in contact.

With such a structure, the amplifier 6 can detect and amplify one of the biological signals, with which the subject is in contact, to obtain an expected effect. Note that, in FIG. 5, the apparatus for detecting biological information 12 is constituted such that the amplifier 6 amplifies a biological signal of one of electrodes only for the electrodes (-) 42 and 82 on the left hand side. However, the apparatus for detecting biological information 12 may be constituted in the same manner by also providing an auxiliary contact piece for the electrode (+) 41 on the right hand side.

In this way, by providing the auxiliary contact piece as a contact member other than the controller, even in the case in which a subject driving an automobile or the like does not grip a main controller such as a control wheel with both hands, if a hand or an arm, which is not in contact with the controller, is in contact with the auxiliary contact piece, biological information of the subject can be detected. Note that, in order to obtain a signal of biological information of the subject, at least a skin of a finger, a hand, an arm, or an elbow is required to be in contact with an electrode serving as the biological information detecting member provided in the contact member.

More specifically, in the apparatuses for detecting biological information 11 and 12 of this embodiment, in the case in which the amplifier 6 amplifies biological signals from the electrodes (41) and (42, 72, or 82) of two lines, the amplifier 6 amplifies a biological signal, which is detected as the subject comes into contact with the electrode 41 provided in the controller 1 of the one line, and a biological signal, which is detected by anyone of the electrode 42 provided in the controller 1 of the other line, the electrode 72 provided in the side brake piece 7, and the electrode 82 provided in the armrest piece 8, with which the subject is in contact.

In addition, in providing an electrode serving as a biological information detecting member in a controller or an auxiliary contact piece of an automobile, a ship, or an airplane, a unit for the electrode comprising a conductive resin layer is attached to the existing controller or auxiliary contact piece, whereby the electrode can be connected to an amplifier serving as an amplifier from this unit. On the other hand, the electrode serving as the biological information detecting member may be obtained by directly coating the paint on the controller or the auxiliary contact piece of the automobile, the ship, or the airplane.

Note that, in the embodiments of the apparatus for detecting biological information shown in FIGS. 4 and 5, since a signal of biological information obtained from one of the controller and the auxiliary contact piece is amplified in the amplifier, an equivalent circuit of the apparatus for detecting biological information is the same as that shown in FIG. 1(b). In addition, the left hand 52 is put on the steering wheel 1 and the side brake piece 7 in FIG. 4, and the left hand 52 is put on the steering wheel 1 and the armrest piece 8 in FIG. 5. However, these figures are for convenience of explanation. Actually, the left hand 52 is put on any one of the steering wheel 1, the side brake piece 7, and the armrest piece 8 or is put on none of the steering wheel 1, the side brake piece 7, and the armrest piece 8.

In the above-described embodiments of the apparatus for detecting biological information, the electrodes 41 and 42 are explained as electrodes comprising the conductive resin layer 45 provided in the control wheel 1. However, the electrodes 41 and 42 are not limited to this and may be any electrodes as long as the electrodes use a material having the predetermined volume resistivity and have the predetermined area of contact with the hands 51 and 52 of the subject of biological information. More specifically, as shown in FIGS. 2 (b) and 2 (c), the steering wheel 2 itself of the control wheel 1 may be an electrode 46 formed of a material having a volume resistivity of 25 $\Omega$cm or less. A position for forming the electrode 46 is not specifically limited. The electrode 46 may be provided only in parts of the steering wheel 2 as shown in FIG. 2(b) or may be provided to on the entire periphery of the steering wheel 2 as shown in FIG. 2 (c). In addition, even in the case in which the electrode 46 is provided in parts of the steering wheel 2, positions are not specifically limited. In this way, the form of the electrodes 41 and 42 is not specifically limited and may be any form as long as the electrodes 41 and 42 can provide the above-described resistance R and electrode impedances r and C.

Note that, in the case in which the electrodes 41 and 42 are formed of the conductive resin layer 45, positions on the electrodes 41 and 42 where paint for the conductive resin layer is applied are not specifically limited. However, usually, as in the above-described embodiments, the paint is applied only on a side in the steering wheel 2 of the control wheel 1 not facing a driver. If the conductive resin layer is formed in this way, the conductive resin layer 45 can accomplish a function as an electrode in an apparatus for detecting biological information without spoiling the looks of the control wheel 1. Note that the electrodes 41 and 42 may be provided only on a side of the steering wheel 2 facing the driver, may be provided only on an outer side or an inner side of the steering wheel 2, or may be provided on the entire outer periphery of the steering wheel 2. However, it is necessary to cause the left and right electrodes 41 and 42 not to come into contact with each other.

In the above-described embodiments of the apparatus for detecting biological information, an electrode is formed of a pair of electrodes 41 and 42. However, the electrode is not limited to this and, for example, may be formed of one electrode, or may be formed of three or more electrodes. In addition, in the same embodiments, the AC differential amplifier 6 is used as the amplifier. However, the amplifier is not limited to this and may be any section having a function of amplifying a potential according to an electrode that detects the potential.

(Example)

Influences of the electrode-amplifier resistance R and the electrode impedances r and C on an electrocardiographic complex will be explained specifically according to an example of the above-described embodiments and comparative examples. Note that, in all cases, the input impedance Z of the amplifier 6 is about 1 MΩ.

(I) Example

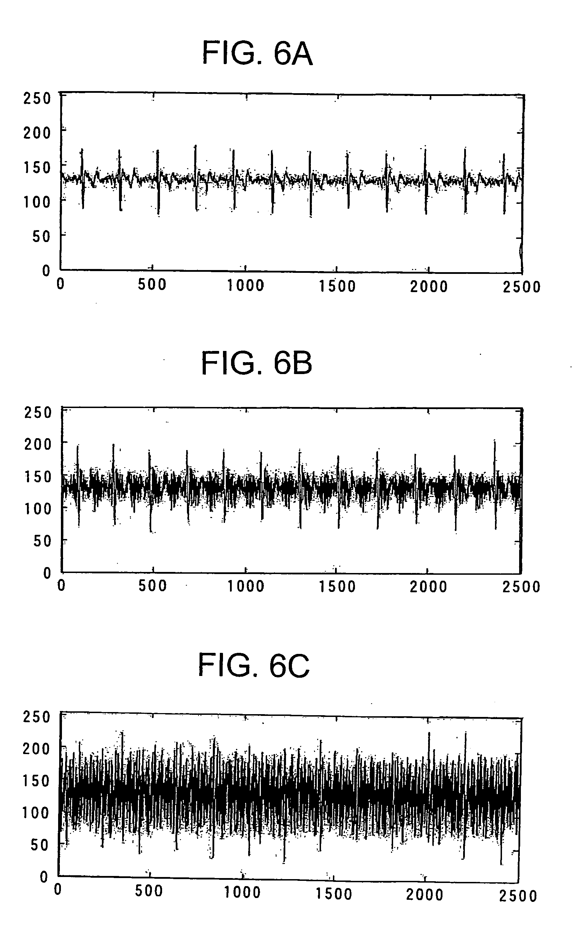

First, an electrocardiographic complex in the case in which the electrode-amplifier resistance R is set to 5 kΩ or less and the electrode impedances r and C are set to 0.4 kΩ is shown in FIG. 6(a). In this case, a sum of the electrode-amplifier resistance R and the electrode impedances r and C is at least 1/100 or less of the input impedance Z of the amplifier 6. In addition, the electrode-amplifier resistance R and the electrode impedances r and C are 1/200 or less of the input impedance Z of the amplifier 6, respectively.

(II) Comparative example 1

Next, an electrocardiographic complex in the case in which the electrode-amplifier resistance R is set to 100 kΩ and the electrode impedances r and C are set to 0.4 kΩ is shown in FIG. 6(b). The electrode-amplifier resistance R is large compared with that defined in the above-described embodiments. In this case, a sum of the electrode-amplifier resistance R and the electrode impedances r and C is 1/100 or more of the input impedance Z of the amplifier 6.

(III) Comparative example 2

Moreover, an electrocardiographic complex in the case in which the electrode-amplifier resistance R is set to 5 kΩ or less and the electrode impedances r and C are set to 10 kΩ is shown in FIG. 6(c). The electrode impedances r and C are large compared with those defined in the above-described embodiments. In this case, a sum of the electrode-amplifier resistance R and the electrode impedances r and C is 1/100 or more of the input impedance Z of the amplifier 6.

(IV) Evaluation result

From FIGS. 6(a) to 6(c), in the case of the example in FIG. 6(a), the electrocardiographic complex appeared clearly, and a potential signal could be detected most stably. On the other hand, in the comparative examples in FIGS. 6 (b) and 6 (c), noise was mixed in the electrocardiographic complex, and the electrocardiographic complex was unclear and was difficult to use.

**Claims**

1. An apparatus for detecting biological information comprising:

   a contact member arranged to come into contact with a subject of biological information;
   a biological information detecting member provided in the contact member and detects the biological information from the subject; and
   an amplifier connected to the biological information detecting member and amplifies a biological signal corresponding to the detected biological information,
   wherein a sum of a resistance between the biological information detecting member and the amplifier, and an impedance between the subject in contact with the contact member and the biological information detecting member, is not more than 1/100 of an input impedance in the amplifier.

2. The apparatus for detecting biological information according to claim 1, wherein a sum of a resistance between the biological information detecting member and the amplifier, and an impedance between the subj ect in contact with the contact member and the biological information detecting member, is not more than 10 kΩ.

3. The apparatus for detecting biological information according to claim 1 or 2, wherein a resistance between the biological information detecting member and the amplifier is not more than 1/200 of an input impedance in the amplifier.

4. The apparatus for detecting biological information according to any one of claims 1 to 3, wherein a resistance between the biological information detecting member and the amplifier is not more than 5 kΩ.

5. The apparatus for detecting biological information according to any one of claims 1 to 4, wherein the biological information detecting member comprises a material having a volume resistivity of not more than 25 Ωcm.

6. The apparatus for detecting biological information according to any one of claims 1 to 5, wherein the biological information detecting member comprises a material containing at least one of silver, nickel, gold, palladium, carbon, and carbon nanotube.

7. The apparatus for detecting biological information according to any one of claims 1 to 5, wherein the biological information detecting member comprises a material containing at least one of metal oxide, which is transparent and has electrical conductivity, and polymer, which is transparent and has electrical conductivity.

8. The apparatus for detecting biological information according to any one of claims 1 to 7, wherein an impedance between the subject in contact with the contact member and the biological information detecting member is not more than 1/200 of an input impedance in the amplifier.

9. The apparatus for detecting biological information according to any one of claims 1 to 8, wherein an impedance between the subject in contact with the contact member and the biological information detecting member is not more than 5 kΩ.

10. The apparatus for detecting biological information according to any one of claims 1 to 9, wherein an area of the biological information detecting member is set so that an area of contact with the subject is not less than 2 cm$^2$ for each place.

11. The apparatus for detecting biological information according to any one of claims 1 to 10, wherein the contact member comprises a controller used for at least one of an automobile, a ship, and an airplane.

12. The apparatus for detecting biological information according to any one of claims 1 to 11, wherein the contact member comprises a controller, used for controlling at least one of an automobile, a ship, and an airplane, and an auxiliary contact piece, constituted to assist a subject controlling at least one of the automobile, the ship, and the airplane using the controller when the subject comes into contact with the auxiliary contact piece.

13. The apparatus for detecting biological information according to claim 12, wherein in the case in which said apparatus for detecting biological information is provided in the automobile, the auxiliary contact piece is at least one of a side brake piece, an armrest piece, and a shift lever piece.

14. The apparatus for detecting biological information according to claim 12 or 13, wherein one of the biological information detecting members provided in the controller, and one of the biological information detecting members provided in the auxiliary contact piece, are connected.

15. The apparatus for detecting biological information according to claim 12 or 13, wherein the amplifier amplifies the biological signal detected by one of the biological information detecting member provided in the controller, and the biological information detecting members provided in the auxiliary contact piece, with which the subject is in contact.

16. The apparatus for detecting biological information according to any one of claims 12 to 15, wherein in the case in which the amplifier amplifies the biological signals from the biological information detecting member of two lines, the amplifier amplifies the biological signal, which is detected when the subject comes into contact with the biological information detecting member provided in the controller of one of two lines, and the biological signal, which is detected by one of the biological information detecting member provided in the controller of another one line of two lines and the biological information detecting member provided in the auxiliary contact piece of said another one of two lines, with which the subject is in contact.

17. The apparatus for detecting biological information according to any one of claims 1 to 16, wherein the biological information detecting member comprises a conductive resin layer provided in the contact member.

18. A contact member that is used as the contact member included in the apparatus for detecting biological information according to any one of claims 1 to 17, wherein
the biological information detecting member has a resistance of not more than 5 kΩ.

19. The contact member according to claim 18, wherein
the biological information detecting member comprises a material having a volume resistivity of not more than 25 Ωcm.

20. The contact member according to claim 18 or 19, wherein the biological information detecting member comprises a material containing at least one of silver, nickel, gold, palladium, carbon, and carbon nanotube.

21. The contact member according to claim 18 or 19, wherein the biological information detecting member comprises a material containing at least one of metal oxide, which is transparent and has electrical conductivity, and polymer, which is transparent and has electrical conductivity.

22. The contact member according to any one of claims 18 to 21, wherein an area of the biological information detecting member is set such that an area of contact with the subject is not less than 2 cm$^2$ for each place.

23. The contact member according to any one of claims 18 to 22, wherein the contact member comprises a controller used for at least one of an automobile, a ship, and an airplane.

24. The contact member according to any one of claims 18 to 23, wherein the contact member comprises a controller, which is used for controlling at least one of an automobile, a ship, and an airplane, and an auxiliary contact piece, which is constituted to assist a subject controlling at least one of the automobile, the ship, and the airplane using the controller when the subject comes into contact with the auxiliary contact piece.

25. The contact member according to claim 24, wherein in the case in which the contact member is provided in the automobile, the auxiliary contact piece is at least one of a side brake piece, an armrest piece, and a shift lever piece.

26. The contact member according to claim 24 or 25, wherein the biological information detecting member provided in the controller and the biological information detecting member provided in the auxiliary contact piece are connected.

27. The contact member according to any one of claims 18 to 26, wherein the biological information detecting member comprises a conductive resin layer provided in the contact member.

28. A paint for a biological information detecting member that constitutes a detecting member for detecting biological information from a subject, wherein
the paint for a biological information detecting member comprises a conductive material having a volume resistivity of not more than 25 Ωcm, epoxy resin, and a curing agent.

29. The paint for a biological information detecting member according to claim 28, wherein the conductive material comprises at least one of silver, nickel, gold, palladium, carbon, and carbon nanotube.

30. The paint for a biological information detecting member according to claim 28, wherein the conductive material comprises least one of metal oxide, which is transparent and has electrical conductivity, and polymer, which is transparent and has electrical conductivity.

## FIG. 1A

## FIG. 1B

# FIG. 2A  FIG. 2B  FIG. 2C

# FIG. 3

# FIG. 4

# FIG. 5

## FIG. 6A

## FIG. 6B

## FIG. 6C

## INTERNATIONAL SEARCH REPORT

| | International application No. |
|---|---|
| | PCT/JP2004/003390 |

**A. CLASSIFICATION OF SUBJECT MATTER**
Int.Cl⁷ A61B5/04, A61B5/0245, B60K28/06

According to International Patent Classification (IPC) or to both national classification and IPC

**B. FIELDS SEARCHED**

Minimum documentation searched (classification system followed by classification symbols)
Int.Cl⁷ A61B5/04, A61B5/0245, B60K28/06, G08B21/06

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | CD-ROM of the specification and drawings annexed to the request of Japanese Utility Model application No. 1272/1992(laid-open No. 58105/1993) (Mitsubishi Motors Corp.), 03 August, 1993 (03.08.93), Full text; all drawings (Family: none) | 1-27 |
| Y | JP 2002-85360 A (Isuzu Motors Ltd.), 26 March, 2002 (26.03.02), Full text; all drawings (Family: none) | 1-27 |

[X] Further documents are listed in the continuation of Box C.     [ ] See patent family annex.

| | |
|---|---|
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 08 June, 2004 (08.06.04) | 22 June, 2004 (22.06.04) |

| Name and mailing address of the ISA/<br>    Japanese Patent Office | Authorized officer |
|---|---|
| Facsimile No. | Telephone No. |

Form PCT/ISA/210 (second sheet) (January 2004)

**INTERNATIONAL SEARCH REPORT**

International application No.

PCT/JP2004/003390

C (Continuation). DOCUMENTS CONSIDERED TO BE RELEVANT

| Category* | Citation of document, with indication, where appropriate, of the relevant passages | Relevant to claim No. |
|---|---|---|
| Y | JP 11-332842 A (Aloka Co., Ltd.), 07 December, 1999 (07.12.99), Par. No. [0019]; Fig. 2 (Family: none) | 1-27 |
| Y | JP 9-168518 A (Casio Computer Co., Ltd.), 30 June, 1997 (30.06.97), Full text; all drawings (Family: none) | 1-27 |
| Y | Microfilm of the specification and drawings annexed to the request of Japanese Utility Model Application No. 11162/1987 (Laid-open No. 120608/1988) (Matsushita Electric Works, Ltd.), 04 August, 1988 (04.08.88), Full text; all drawings (Family: none) | 1-27 |
| Y | Microfilm of the specification and drawings annexed to the request of Japanese Utility Model Application No. 119473/1986 (Laid-open No. 26304/1988) (The Furukawa Electric Co., Ltd.), 20 February, 1988 (20.02.88), Full text; all drawings (Family: none) | 1-27 |
| X | JP 4-168156 A (Tomoegawa Paper Co., Ltd.), 16 June, 1992 (16.06.92), Page 2, lines 9 to 14; page 5, lower left column, lines 3 to 9 (Family: none) | 28-30 |

Form PCT/ISA/210 (continuation of second sheet) (January 2004)

18

## INTERNATIONAL SEARCH REPORT

International application No.

PCT/JP2004/003390

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
   It is not deemed that there is technical relation including one or more identical or corresponding "special technical features" between the invention of a living body information detecting device described in independent Claim 1, the invention of a contact member described in independent Claim 18, and the invention of a living body information detecting member-use paint described in independent Claim 28.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest.

                             ☐ No protest accompanied the payment of additional search fees.

Form PCT/ISA/210 (continuation of first sheet (2)) (January 2004)